(19) Europäisches Patentamt
European Patent Office
Office européen des brevets

(11) **EP 1 289 548 B1**

(12) **EUROPÄISCHE PATENTSCHRIFT**

(45) Veröffentlichungstag und Bekanntmachung des Hinweises auf die Patenterteilung:
**20.02.2008 Patentblatt 2008/08**

(21) Anmeldenummer: **01947157.2**

(22) Anmeldetag: **17.05.2001**

(51) Int Cl.:
**A61K 39/145** *(2006.01)*

(86) Internationale Anmeldenummer:
**PCT/DE2001/001939**

(87) Internationale Veröffentlichungsnummer:
**WO 2001/087333 (22.11.2001 Gazette 2001/47)**

(54) **MITTEL ZUR STIMULATION DES IMMUNSYSTEMS**

IMMUNE SYSTEM STIMULATING MEANS

MOYENS POUR STIMULER LE SYSTEME IMMUNITAIRE

(84) Benannte Vertragsstaaten:
**AT BE CH CY DE DK ES FI FR GB GR IE IT LI LU MC NL PT SE TR**

(30) Priorität: **18.05.2000 DE 10025045**

(43) Veröffentlichungstag der Anmeldung:
**12.03.2003 Patentblatt 2003/11**

(73) Patentinhaber: **Dr. med. Volker von Baehr/ Prof. Dr. med. Rüdiger von Baehr GbR 12257 Berlin (DE)**

(72) Erfinder: **Dr. med. Volker von Baehr/ Prof. Dr. med. Rüdiger von Baehr GbR 12257 Berlin (DE)**

(74) Vertreter: **Maiwald Patentanwalts GmbH Jungfernstieg 38 20354 Hamburg (DE)**

(56) Entgegenhaltungen:
- RODRIGUES MAURICIO ET AL: "Influenza and vaccinia viruses expressing malaria CD8+ T and B cell epitopes: Comparison of their immunogenicity and capacity to induce protective immunity." JOURNAL OF IMMUNOLOGY, Bd. 153, Nr. 10, 1994, Seiten 4636-4648, XP001026182 ISSN: 0022-1767
- MURATA KENICHIRO ET AL: "Characterization of in vivo primary and secondary CD8+ T cell responses induced by recombinant influenza and vaccinia viruses." CELLULAR IMMUNOLOGY, Bd. 173, Nr. 1, 1996, Seiten 96-107, XP001038137 ISSN: 0008-8749
- GLUCK REINHARD: "Adjuvant activity of immunopotentiating reconstituted influenza virosomes (IRIVs)." VACCINE, Bd. 17, Nr. 13-14, 26. März 1999 (1999-03-26), Seiten 1782-1787, XP009158322 ISSN: 0264-410X
- SCHAPIRO J M ET AL: "NATURAL KILLER NK CELL RESPONSE AFTER VACCINATION OF VOLUNTEERS WITH KILLED INFLUENZA VACCINE" JOURNAL OF MEDICAL VIROLOGY, Bd. 30, Nr. 3, 1990, Seiten 196-200, XP001038103 ISSN: 0146-6615
- SAURWEIN-TEISSL M ET AL: "Whole virus influenza vaccine activates dendritic cells (DC) and stimulates cytokine production by peripheral blood mononuclear cells (PBMC) while subunit vaccines support T cell proliferation." CLINICAL AND EXPERIMENTAL IMMUNOLOGY, Bd. 114, Nr. 2, November 1998 (1998-11), Seiten 271-276, XP001038101 ISSN: 0009-9104
- GLUECK REINHARD: "Immunopotentiating reconstituted influenza virosomes (IRIVs) and other adjuvants for improved presentation of small antigens." VACCINE, Bd. 10, Nr. 13, 1992, Seiten 915-919, XP001038106 1st European Conference on Vaccinology;Annecy, France; March 16-17, 1992 ISSN: 0264-410X

EP 1 289 548 B1

**Beschreibung**

Die Erfindung betrifft ein neues Mittel zur unspezifischen Stimulation des Immunsystems und die Verwendung von Proteinen aus Influenzaviren zur Herstellung von Mitteln zur Stimulation des Immunsystems. Dabei werden Proteine des Influenzavirus, wie sie in Influenzaimpfstoffen enthalten sind, verwendet.

Bei dem Influenzavirus - Grippevirus, Myxovirus influenzae - handelt es sich um ein ubiquitäres Orthomyxovirus als Erreger der echten Grippe. Eine aktive Immunisierung gegen Grippe - die Grippeschutzimpfung - erfolgt mit trivalentem Impfstoff aus inaktivierten, auf Hühnerbruteiem gezüchteten Grippeviren oder mit "Subunit"-Impfstoff, der nur noch Hämagglutinin und Neuraminidase des Virus enthält. Die Wirkungsdauer der Grippeschutzimpfung beträgt ein Jahr (Roche-Lexikon Medizin, 3. Auflage - München, Wien, Baltimore - Urban & Schwarzenberg 1993; Lange, W. et al.: Influenza, Blackwell - Berlin - Wien 1999).

Die gegenwärtig angewendeten Grippeimpfstoffe werden nach ihren Typen jährlich dem Epidemiegeschehen angepasst. Das heißt, es müssen pro Jahr jeweils neue Grippeimpfstoffe produziert werden Die lokale und allgemeine Verträglichkeit der inaktivierten Influenzaimpfstoffe ist sehr gut. Die Zielstellung der gegenwärtig durchgeführten Influenzaimpfstoffaktionen im Herbst besteht darin, einen ausreichend hohen Autikörperschutz gegen die zu erwartenden Epidemiestämme hervorzurufen.

Dazu findet jährlich eine Sitzung von Influenzaexperten der Weltgesundheitsorganisation WHO statt, bei der den Impfstoffherstellern die epidemiologisch relevanten Virusstämme für die kommende Saison empfohlen werden. Die WHO stellt den Herstellern das sogenannte Mastervirus zur Verfügung, aus dem dann über das Saatvirus der Impfstoff hergestellt wird (Lange, W. et al.: Influenza, Blackwell - Berlin - Wien 1999).

Vaccine, Band 17, 1999, Seiten 1782-1787 beschreibt die Verwendung von in Form rekonstituierter Influenzavirosomen formuliertem Influenza-Impfstoff zur Erhöhung der spezifischen Immunantwort. J. Immunol., Band 153, 1994, Seiten 4636-4648 sowie Cell. Immunology, Band 173, 1996, Seiten 96-107 beschreiben rekombinante Influenza- und Vaccinia-Viren, die in CD8$^+$T-Zell-Epitope eines Malariaparasiten enthalten, sowie deren kombinierte Anwendung zur Erhöhung antigenspezifischer CD8$^+$T-Zellen.

Hersteller von Influenzaimpfstoffen in Europa sind die Unternehmen Pasteur Merieux MSD / Aventis, Sächsisches Serumwerk Dresden/ SmithKline Beecham, Duphar, Chiron Behring und Solvay Arzneimittel.

Der Erfindung liegt die Aufgabe zugrunde, Mittel zur Verfügung zu stellen, die zur Stimulation von T-Lymphozyten und zur Verwendung bei der Herstellung von Mitteln zur Immunstimulation geeignet sind. Erfindungsgemäß wurde die Aufgabe durch die Verwendung von Proteinen aus Influenzaviren gelöst. Derartige Proteine sind z.B. in Influenzaimpfstoffen enthalten.

Es hat sich überraschenderweise herausgestellt, dass Proteine aus Influenzaviren bei Gesunden, aber auch bei einem großen Teil HIV-Infizierter (HIV - Human Immunodeficiency Virus), in vitro zu starken Stimulationen der Lymphozyten führen und im Rahmen eines Lymphozyten-Stimulationstestes zur Ermittlung der Immunkompetenz von Patienten mit Influenzaimpfstoff die höchsten Stimulationsquoten zu erzielen sind.

Zur Vertiefung und Erklärung dieses Befundes wurden daraufhin molekularbiologische Untersuchungen zum Nachweis der Genexpression für relevante Zytokine - Interleukin 2 (IL2), Interferon-γ (Gammainterferon, IFN-γ) TNF-α (Tumornekrosefaktor-α) - durchgerührt.

Dazu wurden im Markt gebräuchliche Immunstimulantien eingesetzt und mit der Wirkung von Influenzaimpfstoff verglichen. Dabei stellte sich heraus, dass Influenzaimpfstoffe zur weitaus höchsten Stimulation der Genexpression für Zytokine führen.

Darüber hinaus war festzustellen, dass diese Zytokinexpression auch längere Zeit in der Kultur anhält, länger als bei jedem anderen untersuchten Immunstimulans.

Zu den Vergleichsstimulantien zählten Mistelpräparate, Echinacin, Thymusfaktoren und auch ein bis jetzt nur für die Veterinärmedizin zugelassenes Präparat "Baypamun®", das aus attenuierten inaktivierten Pockenviren besteht (Baxevanis, C.N. u.a., Immunopharmacol. 20 (1998), 355-372; Stein, G.M., P.A. Berg, Arzneim.-Forsch. 46 (1996), 635-639; Büssing, A. u.a., Tumor Diagn. Ther. 16 (1995), 49-53; Mayr A. u.a., Tierärztl. Umschau 52 (1997), 3-11).

Erfindungsgemäß werden Influenzaproteine mehrfach appliziert und die jeweiligen Boostereffekte im Sinne einer unspezifischen Immunstimulation genutzt. Mit dieser Immunstimulation kommt es zu einer Verbesserung der Infektresistenz - unabhängig vom Erregertyp - und zur Erhöhung der antitumoralen Reaktion bei Tumorträgern. Für Tumorträger ist dabei entscheidend, dass mit dem Impfstoff auch NK(natural killer)-Zellen stimuliert und aktiviert werden. Ein weiterer Effekt besteht darin, dass eine erhöhte Syntheseaktivität für immunologisch wichtige Zytokine, wie Interleukin 2, Gammainterferon und TNF, induziert wird. Die Einsatzkonzeption für Influenzaimpfstoffe zur unspezifischen Immunstimulation umfasst

- Patienten mit häufigen Infekten, gleichbedeutend mit einer verminderten Infektresistenz;
- Patienten jenseits des 60. Lebensjahres, die erfahrungsgemäß häufiger eine verminderte Leistungsfähigkeit des T-lymphozytären Systems aufweisen - was sich besonders in einer stark verzögerten Rekonvaleszensphase nach Infekten zeigt;
- HIV-Patienten unter antiretroviraler Therapie. Dabei wird die sonst unter Immunstimulation befürchtete Verstärkung der HIV-Replikation durch die antiretrovirale Therapie unterdrückt. Die Immunstimulation dient der Aktivierung von HIV-reaktiven Effektorzellen, die HIV-replizierende Restzellen erkennen und eliminieren sollen;

- Patienten nach schulmedizinischer Tumortherapie ohne nachweisbare Metastasen zur Rezidivprophylaxe bzw. mit nachweisbaren Metastasen zur Erhöhung der antitumoralen Immunreaktivität.

Zum Wirkungsmechanismus der Influenzaproteine als Immunstimulans:

Durch eine wiederholte Applikation des Impfstoffes werden Influenzavirus-spezifische T-Zellen aktiviert und vermehrt. Bei der Aktivierung werden T-Zell-Zytokine wie Interleukin 2 und Interferon gamma freigesetzt. Diese heben in der Umgebung befindliche T-Lymphozyten mit anderen Spezifitäten auf ein höheres Aktivitätsniveau, was besonders für T-Gedächtniszellen zutrifft. Damit wird eine verstärkte Reaktionsbereitschaft gegen andere Viren (HIV, Herpesviren, Hepatitisviren), mit denen der Patient bereits infiziert ist, bzw. exogene Erreger in der Umgebung des Patienten, erreicht.

Dieser Effekt ist jedoch nur zu erzielen, wenn der Influenzaimpfstoff mehrfach appliziert wird. Dabei wird automatisch auch die spezifische Abwehr gegen Influenzavirus verstärkt, die heute mit dem Titeranstieg von Antikörpern gegen das Hämagglutinin des Virus nachgewiesen wird. Für die Anwendung des Immunstimulans ist es jedoch nicht notwendig, jährlich die Zusammensetzung der Impfstämme zu verändern.

Mit Hilfe der vorliegenden Erfindung ist es überraschenderweise möglich, durch Verwendung von Influenzaimpfstoff (Grippeimpfstoff) die Aktivierung einer defizitären Immunabwehr zu erreichen.

Kontraindikationen für diese Maßnahme werden in folgenden Krankheiten gesehen:

- Autoimmunkrankheiten mit Betonung der zellvermittelten Immunität (z.B. Multiple Sklerose, chronische Autoimmunerkrankungen des Darmes wie Mb. Crohn)
- Patienten nach allogener Gewebe- oder Organtransplantation wegen der Gefahr, eine Rejektionskrise zu induzieren.

Das Wesen der Erfindung liegt in der Verwendung eines bekannten Mittels zu einem neuen Zweck und in einer Kombination bekannter Elemente - der Influenzaimpfstoffe - und einer neuen Wirkung - ihrem Einsatz als Mittel zur unspezifischen Immunstimulation - die in ihrer neuen Gesamtwirkung einen Gebrauchsvorteil und den erstrebten Erfolg ergeben, der darin liegt, dass nunmehr ein bewährtes und verbreitetes Arzneimittel als Mittel zur Stimulation von T-Lymphozyten bei einem funktionell gestörten Immunsystem zur Verfügung steht.

Die Erfindung soll anhand von Ausführungsbeispielen näher erläutert werden, ohne auf diese Beispiele beschränkt zu sein.

**Ausführungsbeispiele**

Beispiel 1:

Es wurden von 30 klinisch gesunden Probanden (15 Männer, 15 Frauen - im Alter von 20 bis 55 Jahren) Blutproben entnommen, Immunzellen (Lymphozyten und Monozyten) daraus präpariert und zu je $2 \times 10^5$ Zellen in Mikrokulturen aliquotiert. Zu diesen Mikrokulturen wurden jeweils ein bekanntes Mitogen (Lektine, stimulierende monoklonale Antikörper, Superantigene) und in einer weiteren Reihe von Mikrokulturen Influenzaimpfstoff (Endverdünnung 1 : 1000 einer Impfdosis) hinzugefügt. Die Kulturen wurden in einem serumhaltigen Zellkulturmedium über 6 Tage bei 37°C und 5% $CO_2$-Atmosphäre kultiviert, 6 Stunden vor Abbruch mit $^3$H-Thymidin versetzt und das in die Zell-DNS eingebaute $^3$H-Thymidin mit einem Betacounter quantitativ bestimmt (counts per minute = cpm).

Die Positivkontrolle entspricht der Mitogen-stimulierten Kultur. Die Negativkontrolle enthält kein bekanntes oder potentielles Stimulans.

Das Ergebnis wird als Stimulationsindex (SI) ausgedrückt:

$$SI = \frac{cpm \ (Testansatz)}{cpm \ (Negativkontrolle)}$$

Folgende Ergebnisse wurden erzielt:

| Mitogene - | PHA | SI | 60 +/- 15 |
|---|---|---|---|
| | AntiCD3 | SI | 70+/-20 |
| | Enterotoxin B | SI | 50 +/- 20 |

Immunstimulation

| | Echinacin | SI | < 3 |
|---|---|---|---|
| | Mistellektine | SI | 4 +/- 2,5 |
| | Thymuspeptide | SI | 6 +/- 3 |
| Influsplit- | Impfstoff 1 | SI | 38 +/-14 |
| Influvac- | Impfstoff 2 | SI | 30 /-12 |
| Begrivac- | Impfstoff 3 | SI | 28 +/-14. |

Beispiel 2:

Bei Untersuchungen analog zu Beispiel 1 bei 40 HIV-infizierten Patienten wurden folgende Ergebnisse mit Influenzaimpfstoff erzielt:

Patienten im Stadium I und II

    Influsplit    SI    28 +/-13

Patienten im AIDS-Stadium

    Influsplit    SI    10 +/- 6

Unter einer antiretroviralen Therapie der HIV-Patienten wurde stets ein Anstieg des SI um mehr als 50% gemessen.

Beispiel 3 :

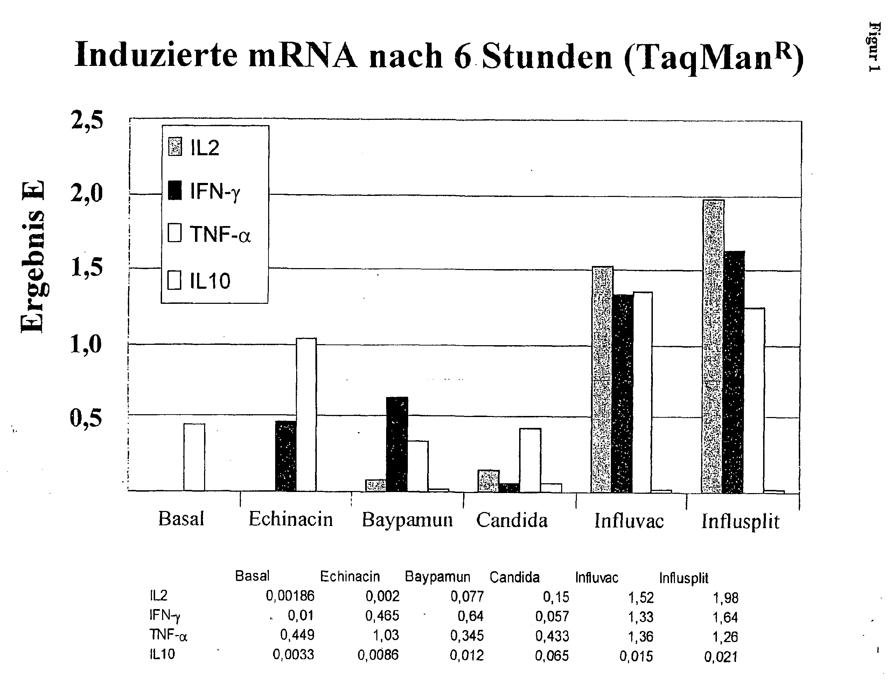

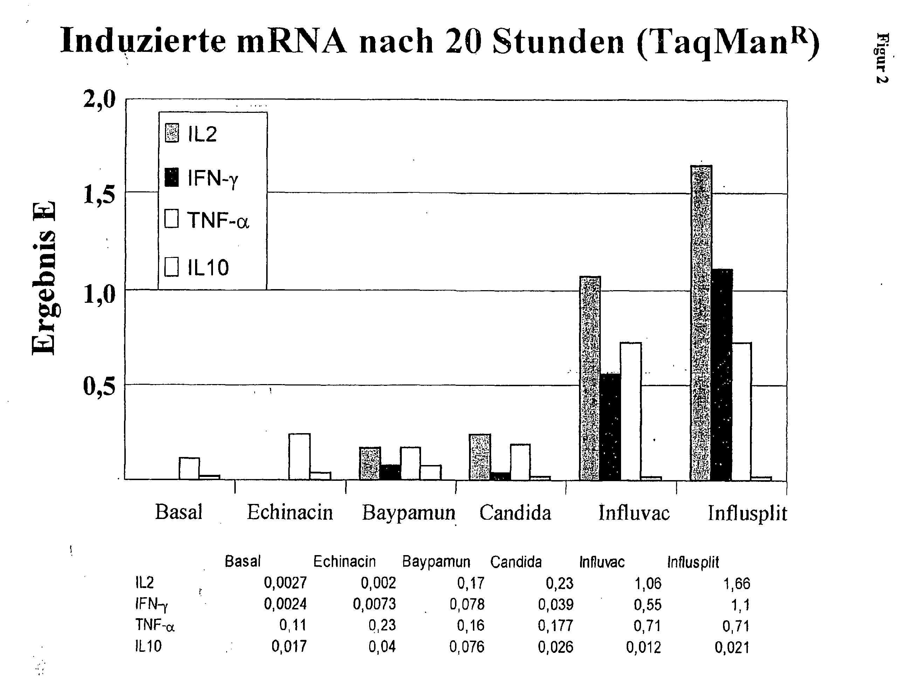

Zur Absicherung der in Beispiel 1 und 2 mittels Lymphozytenstimulationstest dargestellten Aktivierung der T-Lymphozyten durch Influenzaimpfstoffe wurden bei 5 gesunden Probanden Blutproben entnommen, Immunzellen daraus isoliert und analog zu Beispiel 1 mit verschiedenen Immunstimulantien inkubiert. Nach 6 (Figur 1) und nach 20 Stunden (Figur 2) wurde aus den Zellen die mRNA (Messenger-Ribonucleinsäure) nach bekannten Verfahren gewonnen und im TaqMan-System (Fa. Perkin-Elmer) auf den Gehalt an mRNA für Interleukin 2 (IL2), Interferon gamma (IFN-$\gamma$), Tumornekrosefaktor-$\alpha$ (TNF-$\alpha$) und Interleukin 10 (IL10) untersucht.

Die Figuren 1 und 2 zeigen Beispiele einer solchen Untersuchung.

Als Vergleichsstimulatoren wurden Echinacin, Baypamun und Candida neben den Influenzaimpfstoffen Influvac® und Influsplit® untersucht.

Das dargestellte Ergebnis E berechnet sich wie folgt:

$$E = 2^{-\Delta\,Ct}$$

Der Ct-Wert (cycle treshold) stellt die PCR-Zykluszahl (PCR - polymerase chain reaction) dar, bei der das gemessene Signal des Zytokins (korreliert zur Menge des induzierten Zytokins) erstmals das 10-fache der Standardabweichung der Null-Linie (d.h. des Hintergrundrauschens) übersteigt, also ein eindeutiges Signal erkennbar ist.

Der $\Delta$Ct-Wert (delta cycle treshold) bedeutet: Ct-Wert des jeweiligen Zytokins - (minus) Ct-Wert eines House-Keeping Gens, z.B. HPRT (Hypoxantin-Ribosyl-Transferase).

Ein House-Keeping Gen ist das Gen eines Proteins, das durch Stimulanz selbst nicht reguliert wird, z.B. ein Strukturprotein aus der Zellwand. Das bedeutet, dass aus der Signalhöhe dieses Gens direkt auf die Gesamt-mRNA-Menge, letztlich die Gesamt-Zellzahl im Ansatz, geschlossen werden kann.

Beide Influenzaimpfstoffe stimulieren die mRNA-Synthese für Interleukin 2 (T-Zellwachstumsfaktor), Interferon gamma (NK-Zellstimulator) und TNF-$\alpha$ (Phagozytenstimulator) ohne gleichzeitige Aktivierung des immunsuppressiv wirkenden Interleukin 10.

Damit wurde mit einer unabhängigen Methode die stark T-Lymphozyten-stimulierende Wirkung von Influenzaproteinen bestätigt.

**Patentansprüche**

1. Verwendung von Influenzavirusproteinen, wie sie in Influenzaimpfstoffen enthalten sind, zur Herstellung eines Arzneimittels zur Behandlung von Herpes und/oder Hepatitis und/oder HIV und/oder für die Tumortherapie, wobei das Arzneimittel für die Mehrfachapplikation der Influenzavirusproteine vorgesehen ist.

2. Verwendung gemäß Anspruch 1, **dadurch gekennzeichnet, dass** die Influenzavirusproteine Hämagglutinin und/oder Neuraminidase sind.

**Claims**

1. Use of influenza virus proteins, as contained in influenza vaccines, for the preparation of a medicinal product for the treatment of herpes and/or hepatitis and/or HIV and/or for tumour therapy, wherein the medicinal product is intended for the multiple application of the influenza virus proteins.

2. Use according to claim 1, **characterised in that** the influenza virus proteins are haemagglutinin and/or neuraminidase.

**Revendications**

1. Utilisation de protéines du virus de la grippe contenues dans des vaccins contre la grippe pour la préparation d'un médicament destiné à traiter l'herpès et/ou l'hépatite et/ou le VIH et/ou à traiter des tumeurs, le médicament étant prévu pour l'application multiple des protéines du virus de la grippe.

2. Utilisation selon la revendication 1, **caractérisée en ce que** les protéines du virus de la grippe sont l'hémagglutinine et/ou la neuraminidase.

Figur 1

EP 1 289 548 B1

# Induzierte mRNA nach 6 Stunden (TaqMan$^R$)

|  | Basal | Echinacin | Baypamun | Candida | Influvac | Influsplit |
|---|---|---|---|---|---|---|
| IL2 | 0,00186 | 0,002 | 0,077 | 0,15 | 1,52 | 1,98 |
| IFN-γ | 0,01 | 0,465 | 0,64 | 0,057 | 1,33 | 1,64 |
| TNF-α | 0,449 | 1,03 | 0,345 | 0,433 | 1,36 | 1,26 |
| IL10 | 0,0033 | 0,0086 | 0,012 | 0,065 | 0,015 | 0,021 |

# Induzierte mRNA nach 20 Stunden (TaqMan$^R$)

Figur 2

EP 1 289 548 B1

| | Basal | Echinacin | Baypamun | Candida | Influvac | Influsplit |
|---|---|---|---|---|---|---|
| IL2 | 0,0027 | 0,002 | 0,17 | 0,23 | 1,06 | 1,66 |
| IFN-$\gamma$ | 0,0024 | 0,0073 | 0,078 | 0,039 | 0,55 | 1,1 |
| TNF-$\alpha$ | 0,11 | 0,23 | 0,16 | 0,177 | 0,71 | 0,71 |
| IL10 | 0,017 | 0,04 | 0,076 | 0,026 | 0,012 | 0,021 |

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- Roche-Lexikon Medizin. Urban & Schwarzenberg, 1993, vol. 3
- **LANGE, W. et al.** Influenza. Blackwell, 1999
- *Vaccine,* 1999, vol. 17, 1782-1787
- *J. Immunol.,* 1994, vol. 153, 4636-4648
- *Cell. Immunology,* 1996, vol. 173, 96-107
- **BAXEVANIS, C.N.** *Immunopharmacol,* 1998, vol. 20, 355-372
- **STEIN, G.M. ; P.A. BERG.** *Arzneim.-Forsch.,* 1996, vol. 46, 635-639
- **BÜSSING, A.** *Tumor Diagn. Ther.,* 1995, vol. 16, 49-53
- **MAYR A.** *Tierärztl. Umschau,* 1997, vol. 52, 3-11